# EUROPÄISCHE PATENTSCHRIFT

(11) **EP 1 134 223 B1**
(45) Veröffentlichungstag und Bekanntmachung des Hinweises auf die Patenterteilung: **26.10.2005**
(21) Anmeldenummer: 01105396.4
(22) Anmeldetag: 12.03.2001
(51) Int. Cl.: C07F 1/00, C07F 3/00, A23L 1/236, C07F 13/00, C07F 15/02, C07F 15/06

(54) **Acesulfam-Metall-Komplexe, Verfahren zu ihrer Herstellung und ihre Verwendung**
Acesulfam metal complexes, their preparation and use
Complexes métalliques d'acesulfame, leur préparation et utilisation

(30) Priorität: 17.03.2000 DE 10013259
(43) Veröffentlichungstag der Anmeldung: 19.09.2001
(73) Patentinhaber: Nutrinova Nutrition Specialties & Food Ingredients GmbH, 65929 Frankfurt am Main (DE)
(72) Erfinder: Burgard, Andreas, Dr., 65929 Frankfurt am Main (DE)
(74) Vertreter: Schweitzer, Klaus

(56) Entgegenhaltungen:
- WO-A-00/72701
- BECK, W. ET AL.: "Palladium- und Platin-komplexe mit den Anionen von 6-Methyl-1,2,3-oxathiazin-4(3H)-on-2,2-dio xid und N-2-Pyrimidinylsulfanilamid" CHEMISCHE BERICHTE, Bd. 118, Nr. 2, 1985, Seiten 444-449, XP002204730

## Beschreibung

Einige Spurenelemente sind für die normalen Körperfunktionen wichtig. Bei Mangelerscheinungen ist die gezielte Zufuhr von Spurenelementen über die mit der üblichen Ernährung aufgenommenen Mengen hinaus erforderlich oder mindestens wünschenswert. Derartige Mangelerscheinungen können bei falscher Ernährung, aber auch bei gestörter Aufnahme infolge von Fehlfunktionen des Körpers eintreten. Die ausreichende Versorgung mit Spurenelementen ist nicht allein beim Menschen, sondern auch in der Tiermast wichtig. Spurenelemente und ihre Bedeutung sind z.B. in Römpp Chemie Lexikon, 9. Auflage, unter diesem Stichwort beschrieben. Zu diesen Spurenelementen gehören insbesondere Eisen, Zink, Kupfer, Mangan und Kobalt.

Problematisch für die ausreichende Versorgung mit Spurenelementen, insbesondere in Form ihrer Salze, ist dabei allerdings, daß viele Salze dieser Spurenelemente bereits in geringen Mengen unangenehm schmecken und insbesondere einen adstringierenden Geschmack aufweisen. Die Einarbeitung von Verbindungen dieser Spurenelemente in geeignete Lebensmittel, Arzneimittel oder Futtermittel und die Zufuhr über spezielle Präparate kann deshalb aufgrund mangelnder Akzeptanz durch Mensch und Tier schwierig sein. Die Bereitstellung in einer angenehm schmeckenden Form würde die Akzeptanz und die Möglichkeiten der gezielten Anwendung in Lebensmitteln, Arznei- und Futtermitteln deutlich verbessern.

In CHEMISCHE BERICHTE, Bd. 118, Nr.2, 1985, S. 444-449 (XP002204730) werden von Beck et al. Palladium- und Platinkomplexe mit den Anionen von 6-Methyl-1,2,3-oxathiazin-4(3H)-on-2,2-dioxid und N-2-Pyrimidinylsulfanilamid beschrieben.

Es wurde nun gefunden, daß der bekannte Süßstoff Acesulfam (6-Methyl-3,4-dihydro-1,2,3-oxathiazin-4-on-2,2-dioxid), der bisher nur als Kaliumsalz(Acesulfam-K) angeboten wird und von dem Salze mit Alkali- und Erdalkalimetallen, z.B. Natrium-, Kalium-, Magnesium- und Calciumsalze, das sauer reagierende Acesulfam selbst, das die sogenannte Acesulfamsäure darstellt, und einige Aminsalze bekannt sind, mit diesen Elementen stabile Komplexe bevorzugt mit jeweils zwei Acesulfam-Anionen je Metallatom bildet. Diese Komplexe zeichnen sich überraschenderweise durch einen angenehm süßen Geschmack aus, dem die adstringierende Note vieler Salze dieser Spurenelemente fehlt.

Die vorliegende Erfindung umfaßt somit Komplexverbindungen der Spurenelemente Zink, Kupfer, Eisen, Mangan und Kobalt mit Acesulfam. Es handelt sich dabei bevorzugt um Metallkomplexe des Acesulfams, die aus einem Spurenelementmetall und 2 Acesulfam-Molekülen zusammengesetzt sind und die darüber hinaus gegebenenfalls Kristallwasser enthalten können. Es handelt sich um definierte Verbindungen, bei denen das Metall kationisch und die Acesulfammoleküle anionisch vorliegen.

Dadurch, daß die Spurenelemente bzw. Metallkationen in diesen Komplexen bevorzugt salzartig gebunden sind, sind sie nach Solvolyse durch Wasser bioverfügbar und können vom Stoffwechsel ohne weiteres aufgenommen werden.

Diese Komplexe können in den gängigen Verarbeitungsschritten von Lebensmitteln und der Herstellung von Präparaten zur Ergänzung der Nahrung, oder in Arzneimitteln und kosmetischen Mitteln z.B. Zahnpflege- und Mundpflegemittel, oder in Futtermitteln oder als Futtermittelzusatzstoffe, z.B. in Form eines sog. Prä-mix problemlos eingesetzt werden. Die Verarbeitung ist einfach und erfolgt nach bekannten Methoden. Bei festen Zubereitungen werden die erfindungsgemäßen Komplexe in fester Form, ggf. in geeigneter Korngröße, mit den anderen Zutaten vermischt. Zur Verwendung in Tabletten, Komprimaten und vergleichbaren Produkten sowie pulverförmigen Zubereitungen können sie mit anderen dafür geeigneten Zutaten granuliert und als Granulat weiterverarbeitet werden. Auf Grund ihrer guten Löslichkeit können sie aber auch leicht in flüssigen Produkten aus den genannten Bereichen eingesetzt oder in Form ihrer wäßrigen Lösungen verarbeitet werden.

Die vorliegende Erfindung umfaßt somit auch die Verwendung der erfindungsgemäßen Acesulfam-Spurenelementkomplexe als Nahrungsergänzungsmittel oder Lebensmittelzusatzstoff und deren Verwendung in Arznei-, Futter- oder kosmetischen Mittel, wie z.B. Zahnpflege- oder Mundpflegemittel, in jeder dafür geeigneten Form wie z.B. als feste Präparationen in Form von z.B. Tabletten, Kapseln, Komprimaten, Granulaten, fester Prä-mix, pulverförmigen Zubereitungen oder als flüssige Präparationen wie z.B. Lösungen, bevorzugt wäßrige Lösungen oder flüssiger Prä-mix.

Schließlich betrifft die vorliegende Erfindung auch ein Verfahren zur Herstellung der erfindungsgemäßen Komplexverbindungen. Das als Ausgangssubstanz dienende Acesulfam oder dessen Kaliumsalz, Acesulfam-K, sind im Handel erhältlich oder können, wie auch beliebige andere Acesulfamsalze, nach dem in der EP-A 0 155 634 beschriebenen Verfahren hergestellt werden.

Zur Herstellung dieser Komplexe wird ein Verfahren angewandt, mit dem sich andere ionische Bestandteile der für die Herstellung geeigneten Ausgangsstoffe beseitigen lassen. Das kann entweder durch Abtrennung von schwerlöslichen Verbindungen der anderen ionischen Bestandteile oder durch den Einsatz von Ausgangsmaterialien geschehen, mit denen von vornherein nur Spurenelemente und Acesulfam in einer Lösung verbleiben, aus der die Komplexe in geeigneter Weise isoliert werden. Zu diesem Verfahren gehören
- Die Umsetzung von Salzen des Acesulfams, deren Kationen geeignete schwerlösliche Verbindungen bilden, die sich ausfällen lassen wie insbesondere das Calcium-, aber auch das Bariumsalz des Acesulfams, mit löslichen Salzen der Spurenelemente, deren Anionen mit den Kationen des Acesulfamsalzes schwerlösliche Verbindungen bilden, z. B. Sulfate, oder
- Die Umsetzung von basischen Carbonaten der Spurenelemente mit Acesulfamsäure(Acesulfam) unter Freisetzung von CO₂
wobei jeweils gebildete Niederschläge gegebenenfalls abgetrennt werden, bevor die gewünschten Acesulfam-Komplexe isoliert werden. Diese Isolierung erfolgt bevorzugt durch Kristallisation, z.B. durch Verdampfen des Lösungsmittels, bevorzugt Wasser oder mit Wasser mischbare Lösungsmittel, oder durch Zugabe von mit Wasser mischbaren Lösungsmitteln zum Reaktionsgemisch. Bevorzugte mit Wasser mischbare Lösungsmittel sind z.B. Alkohole.

Die als Ausgangsstoffe der Reaktion dienenden Salze des Acesulfams können z.B. als wässrige Lösung vorgelegt oder aber auch in einer sog. Eintopfreaktion, vor Zugabe des Spurenelementsalzes, aus Acesulfam und einem geeigneten Erdalkalicarbonat (Ba, Ca-Salz) gebildet werden.

Die Erfindung wird durch die folgenden Beispiele näher erläutert, ohne dadurch ihren Umfang zu beschränken:

Die Erfindung wird durch die folgenden Beispiele erläutert:

### Beispiel 1

### Acesulfam-Zink-Komplex:

### Methode 1:

20 mmol (3,947 g) schwerlösliches Bariumcarbonat (oder 20 mmol Calciumcarbonat) werden in 20 ml Wasser vorgelegt und mit 40 mmol (6,525 g) Acesulfam-H versetzt. Nach Ende der CO₂-Entwicklung erhält man eine homogene Lösung, aus der mit 20 mmol (0,575 g) Zink(II)sulfat-heptahydrat schwerlösliches Bariumsulfat (oder Calciumsulfat) ausgefällt wird. Nach Abfiltrieren des Niederschlags und Einengen der Lösung kristallisiert mit 97 % Ausbeute der Acesulfam-Zink-Komplex in Form farbloser Kristalle aus.

### Methode 2:

10 mmol (3,42 g) schwerlösliches basisches Zinkcarbonat-hydrat (ZnCO₃ x 2 Zn(OH)₂ x H₂O) werden in 20 ml Wasser vorgelegt und mit 60 mmol (9,789 g) Acesulfam-H versetzt. Nach Ende der CO₂-Entwicklung erhält man eine homogene Lösung. Der Acesulfam-Zink-Komplex kristallisiert mit 99 % Ausbeute nach Einengen der Lösung in Form farbloser Kristalle aus.

Der Acesulfam-Zink-Komplex zersetzt sich bei 255°C.

Die Kristallstruktur des Acesulfam-Zink-Komplexes konnte durch eine Röntgenstrukturanalyse aufgeklärt werden. 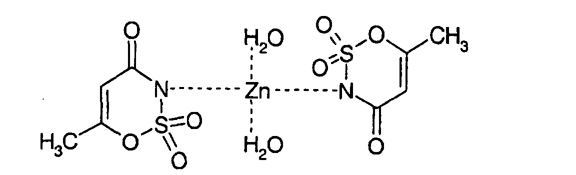 (Ace)₂Zn, 2 C₄H₄NO₄S * Zn * 2 H₂O, M, = 425.69, monoclinic, C2/c, a = 12.907(4), b = 5.584(2), c = 21.222(8) Å, β = 91.31(3)°, V =1529.2(8) Å³, Z = 4, Dₓ = 1.849 Mg m⁻³, λ (Mo-Kα) = 0.71073 Å, µ = 1.932 mm⁻¹, F(000) = 864, T = 293(2) K, R = 0.0235 and R_{w} = 0.0649 for I > 2σ(I) (1356 reflections), R = 0.0255 and R_{w} = 0.0669 for all 1436 unique CCD data. Σ(Fₒ² - F_{c}²)² was minimized.

### Beispiel 2

### Acesulfam-Kupfer-Komplex:

### Methode 1:

20 mmol (3,947 g) schwerlösliches Bariumcarbonat (oder 20 mmol Calciumcarbonat) werden in 20 ml Wasser vorgelegt und mit 40 mmol (6,525 g) Acesulfam-H versetzt. Nach Ende der CO₂-Entwicklung erhält man eine homogene Lösung, aus der mit 20 mmol (0,499 g) Kupfer(II)sulfat-pentahydrat schwerlösliches Bariumsulfat (oder Calciumsulfat) ausgefällt wird. Nach Abfiltrieren des Niederschlags und Einengen der Lösung kristallisiert der Acesulfam-Kupfer-Komplex mit 96 % Ausbeute in Form blauer Kristalle aus.

### Methode 2:

10 mmol (2,21 g) schwerlösliches basisches Kupfercarbonat (CuCO₃ x Cu(OH)₂) werden in 20 ml Wasser vorgelegt und mit 40 mmol (6,526 g) Acesulfam-H versetzt.

Nach Ende der CO₂-Entwicklung erhält man eine homogene blaue Lösung. Der Acesulfam-Kupfer-Komplex kristallisiert mit 98 % Ausbeute nach Einengen der Lösung in Form blauer Kristalle aus.

Der blaugefärbte Acesulfam-Kupfer-Komplex entfärbt sich bei 117°C und zersetzt sich bei 179°C.

Die Kristallstruktur des Acesulfam-Kupfer-Komplexes konnte durch eine Röntgenstruktur-analyse aufgeklärt werden. 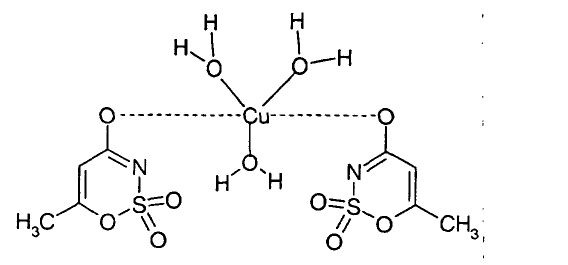 (Ace)₂Cu, 2 C₄H₄NO₄S * Cu * 3 H₂O, M, = 441.87, monoclinic, C2/c, a = 19.30(2), b = 9.677(9), c = 9.007(8) Å, β = 102.92(2)°, V =1640(3) Å³, Z = 4, Dₓ = 1.790 Mg m⁻³, λ (Mo-Kα) = 0.71073 Å, µ = 1.645 mm⁻¹, F(000) = 900, T = 293(2) K, R = 0.0371 and R_{w} = 0.0753 for I > 2σ(I) (1251 reflections), R = 0.0440 and R_{w} = 0.0791 for all 1448 unique CCD data. Σ(Fₒ² - F_{c}²)² was minimized.

In vergleichbarer Weise lassen sich auch die anderen Acesulfam-Spurenelementmetall-Komplexe wie z.B. Mangan-, Kobalt- und Eisen-Acesulfam-Komplexe herstellen.

## Patentansprüche

1. Verbindung aus einem Spurenelement, Acesulfam und gegebenenfalls Kristallwasser, **dadurch gekennzeichnet, daß** es sich bei dem Spurenelement um Zink, Kupfer, Eisen, Mangan oder Kobalt handelt.

2. Verbindung nach Anspruch 1, **dadurch gekennzeichnet, daß** es sich um einen Acesulfam-Metall-Komplex handelt, dessen Stöchiometrie Metall : Acesulfam gleich 1 : 2 ist.

3. Verbindung nach Anspruch 1 oder 2, **dadurch gekennzeichnet, daß** es sich um eine ionische Verbindung handelt.

4. Verfahren zur Herstellung einer Verbindung nach einem oder mehreren der Ansprüche 1 bis 3
a) durch Umsetzung eines in einem geeigneten Lösungsmittel löslichen Acesulfamsalzes mit einem in diesem Lösungsmittel ebenfalls löslichen Salz eines Spurenelements, wobei das Kation des Acesulfamsalzes mit dem Anion des Salzes des Spurenelements eine in diesem Lösungsmittel schwerlösliche Verbindung bildet, Abtrennung dieser Verbindung von dem in Lösung verbliebenen Acesulfam-Metall-Komplex und gegebenenfalls Isolierung dieses Komplexes, oder
b) durch Umsetzung in einem geeigneten Lösungsmittel von Carbonaten, bevorzugt basischen Carbonaten, der Spurenelemente mit Acesulfamsäure und gegebenenfalls Isolierung des gebildeten Acesulfam-Metall-Komplexes,
wobei es sich bei dem Spurenelement um Zink, Kupfer, Eisen, Mangan oder Kobalt handelt.

5. Verfahren nach Anspruch 4, **dadurch gekennzeichnet, daß** bei der Variante a) Carbonate, bevorzugt Barium- oder Calciumcarbonat, zur Herstellung des Acesulfamsalzes aus Acesulfamsäure verwendet werden.

6. Verfahren nach Anspruch 4 oder 5, **dadurch gekennzeichnet, daß** als Salze der Spurenelemente Sulfate verwendet werden.

7. Verfahren nach einem oder mehreren der Ansprüche 4 bis 6, **dadurch gekennzeichnet, daß** als Lösungsmittel Wasser und/oder mit Wasser mischbare Lösungsmittel verwendet werden.

8. Verwendung einer Verbindung nach einem oder mehreren der Ansprüche 1 bis 3 als Süßstoff, Nahrungsergänzungsmittel, Lebensmittel-, Arzneimittel- oder Futtermittelzusatzstoff oder als Zusatzstoff für kosmetische Mittel.

9. Zubereitung enthaltend eine Verbindung nach einem oder mehreren der Ansprüche 1 bis 3.

10. Zubereitung nach Anspruch 9, **dadurch gekennzeichnet, daß** es sich um eine Tablette, Granulat, Komprimat, pulverförmige oder flüssige Zubereitung oder einen Prä-mix handelt.

## Claims

1. A compound of a trace element, acesulfame with or without water of crystallization, wherein the trace element is zinc, copper, iron, manganese or cobalt.

2. A compound as claimed in claim 1 that is an acesulfame-metal complex whose stoichiometry metal:acesulfame is equal to 1:2.

3. A compound as claimed in claim 1 or 2 that is an ionic compound.

4. A process for preparing a compound as claimed in one or more of claims 1 to 3
a) by reacting an acesulfame salt which is soluble in a suitable solvent with a trace element salt which is also soluble in this solvent, the cation of the acesulfame salt forming a compound with the anion of the trace element salt, which compound is sparingly soluble in this solvent, separating off this compound from the acesulfame-metal complex which remains in solution and if appropriate isolating this complex, or
b) by reacting carbonates, preferably basic carbonates, of the trace elements with acesulfamic acid in a suitable solvent and if appropriate isolating the acesulfame-metal complex formed, wherein the trace element is zinc, copper, iron, manganese or cobalt.

5. The process as claimed in claim 4, wherein, in variant a), carbonates, preferably barium carbonate or calcium carbonate, are used for preparing the acesulfame salt from acesulfamic acid.

6. The process as claimed in claim 4 or 5, wherein the trace element salts used are sulfates.

7. The process as claimed in one or more of claims 5 to 6, wherein the solvent used is water and/or water-miscible solvents.

8. The use of a compound as claimed in one or more of claims 1 to 3 as sweetener, food supplement, food additive, medicament additive or feed additive or as an additive for cosmetic compositions.

9. A preparation comprising a compound as claimed in one or more of claims 1 to 3.

10. The preparation as claimed in claim 9 that is a tablet, granules, a compressed composition, pulverulent or liquid preparation or a premix.

## Revendications

1. Composé constitué d'un oligo-élément, d'acésulfame et éventuellement d'eau de cristallisation, **caractérisé en ce que** l'oligo-élément est le zinc, le cuivre, le fer, le manganèse ou le cobalt.

2. Composé selon la revendication 1, **caractérisé en ce qu'**il s'agit d'un complexe métallique d'acésulfame, dont la stoechiométrie métal : acésulfame est de 1 : 2.

3. Composé selon les revendications 1 ou 2, **caractérisé en ce qu'**il s'agit d'un composé ionique.

4. Procédé pour la production d'un composé selon une ou plusieurs des revendications 1 à 3
a) Par la réaction d'un sel d'acésulfame soluble dans un solvant approprié avec un sel d'un oligo-élément lui aussi soluble dans le même solvant, où le cation du sel d'acésulfame forme avec l'anion de l'oligo-élément un composé difficilement soluble dans ce solvant, la séparation de ce composé du complexe métallique d'acésulfame restant en solution et l'isolation éventuelle de ce complexe, ou
b) Par la réaction de carbonates des oligo-éléments, de préférence des carbonates basiques, dans un solvant approprié avec de l'acide acésulfamique et isolation éventuelle du complexe métallique d'acésulfame formé,
où l'oligo-élément est le zinc, le cuivre, le fer, le manganèse ou le cobalt.

5. Procédé selon la revendication 4, **caractérisé en ce que** pour la variante a) on utilise des carbonates, de préférence du carbonate de baryum ou de calcium, pour la production du sel d'acésulfame à partir de l'acide acésulfamique.

6. Procédé selon la revendication 4 ou 5, **caractérisé en ce qu'**on utilise des sulfates comme sels de l'oligo-élément.

7. Procédé selon une ou plusieurs des revendications 4 à 6, **caractérisé en ce qu'**on utilise comme solvant de l'eau et/ou des solvants miscibles dans l'eau.

8. Utilisation d'un composé selon une ou plusieurs des revendications 1 à 3 comme édulcorant, comme complément alimentaire, comme additif pour produits alimentaires, comme additif pour médicaments, comme additif pour fourrage, ou comme additif pour produits cosmétiques.

9. Préparation contenant un composé selon une ou plusieurs des revendications 1 à 3.

10. Préparation selon la revendication 9, **caractérisée en ce qu'**il s'agit d'une pastille, de granulats, de comprimés, d'une préparation en poudre ou liquide ou d'un prémélange.
